# EUROPEAN PATENT APPLICATION

(11) **EP 3 072 486 A1**
(43) Date of publication of application: **28.09.2016**
(21) Application number: 15160837.9
(22) Date of filing: 25.03.2015
(51) Int. Cl.: A61F 13/494, A61F 13/495

(54) **ABSORBENT ARTICLE WITH IMPROVED DESIGN**

(71) Applicant: The Procter & Gamble Company, Cincinnati, OH 45202 (US)
(72) Inventor: Martynus, Cornelia Beate, 65824 Schwalbach (DE); Roe, Donald Carroll, Cincinnati, OH Ohio 45202 (US)
(74) Representative: Borbach, Markus

(57) **Abstract**

The invention relates to an infant or adult diaper. More particularly, the invention relates to an infant or adult diaper (10) having a longitudinal centerline (Y) and a lateral centerline (X) perpendicular to the longitudinal centerline (Y), wherein the diaper (10) comprises a topsheet (22) and a backsheet (24) and positioned therein between an absorbent core (20), and a first longitudinally extending elastic cuff (30a) and second longitudinally extending elastic cuff (30b), the first and the second elastic cuff (30a, 30b) being positioned on either longitudinal side of the topsheet (22) and opposing each other, and wherein the diaper (10) has a crotch region (B), positioned in between a front region (A) and a back region (C) and the first and the second longitudinally extending elastic cuff (30a, 30b) each have a crotch region (B'), positioned in between a front region (A') and a back region (C'), these regions of the longitudinally extending elastic cuffs (30a, 30b) being positioned in the respective regions of the diaper (10), and the first longitudinally extending elastic cuff (30a) and the second longitudinally extending elastic cuff (30b) each have a proximal edge (32a, 32b), at which the respective longitudinally extending elastic cuff is attached to the diaper and the first longitudinally extending elastic cuff (30a) and the second longitudinally extending elastic cuff (30b) each have a distal edge (34a, 34b), at least the crotch region (B') of which is not attached to the diaper (10) and forms a free flap (38a, 38b) and wherein the diaper (10) comprises a barrier element (70) arranged in the crotch region (B), the barrier element (70) comprising at least a wall element (76) and bridge element (72), the barrier element (70) extending in three dimensions such that it has a shape in the longitudinal plane of the diaper, the shape being symmetrical about at least one transversal plane..

## Description

### FIELD OF THE INVENTION

This invention is directed to diapers, including infant diapers, training pants, and adult incontinence articles, and the like having an improved fit, leakage protection and storage behaviour. In one aspect, an improved fit, leakage protection and storage behaviour are achieved by using a barrier element. The present inventions seeks to provide a respective diaper design in such a way that it is easy to manufacture.

### BACKGROUND OF THE INVENTION

Several diaper types have been proposed with components to improve fit and / or reduce leakage of feces and urine from the diaper, to reduce soiling of the genitals or other skin by the feces, or to reduce mixing of urine and feces, to further reduce the risk of irritation of the skin. For example, diapers with a topsheet with an opening, providing a passageway to a void space for collected feces and urine have been proposed; also proposed are diapers with two openings to receive the urine and feces in separate areas; also proposed are diapers with a transversely positioned three-dimensional resilient barrier wall or partition placed in the centre of the diaper, to receive feces and urine respectively on either side of said wall or partition, and to avoid migration of the feces to the front of the article.

For example, US 2011/0015606 (Nakajima et al.) discloses an absorbent article with a liquid-absorbent structur and with a barrier sheet provided in the crotch zone, the barrier sheet comprising a front and a rear through-hole, wherein a void space is formed between the barrier sheet and the liquid absorbent structure.

While the barrier sheet seems to shield excrements from the skin of a wearer, excrement is still not kept in a small defined zone of the diaper. The construction also appears costly and complex.

The inventors found, however that certain diaper design allow for a more efficient containment of excrement in a defined space. While the design is more efficient for this purpose it is not complex or costly. The design also allows for an efficient manufacturing process.

### SUMMARY OF THE INVENTION

The invention relates to an infant or adult diaper. More particularly, the invention relates to a diaper having a longitudinal centerline (Y) and a lateral centerline (X) perpendicular to the longitudinal centerline (Y), wherein the diaper (10) comprises a topsheet (22) and a backsheet (24) and positioned therein between an absorbent core (20), and a first longitudinally extending elastic cuff (30a) and second longitudinally extending elastic cuff (30b), the first and the second elastic cuff (30a, 30b) being positioned on either longitudinal side of the topsheet (22) and opposing each other, and wherein the diaper (10) has a crotch region (B), positioned in between a front region (A) and a back region (C) and the first and the second longitudinally extending elastic cuff (30a, 30b) each have a crotch region (B'), positioned in between a front region (A') and a back region (C'), these regions of the longitudinally extending elastic cuffs (30a, 30b) being positioned in the respective regions of the diaper (10), and the first longitudinally extending elastic cuff (30a) and the second longitudinally extending elastic cuff (30b) each have a proximal edge (32a, 32b), at which the respective longitudinally extending elastic cuff is attached to the diaper and the first longitudinally extending elastic cuff (30a) and the second longitudinally extending elastic cuff (30b) each have a distal edge (34a, 34b), at least the crotch region (B') of which is not attached to the diaper (10) and forms a free flap (38a, 38b) and wherein the diaper (10) comprises a barrier element (70) arranged in the crotch region (B), the barrier element (70) comprising at least a wall element (76) and bridge element (72), the barrier element (70) extending in three dimensions such that it has a shape in the longitudinal plane of the diaper, the shape being symmetrical about at least one transversal plane.

### BRIEF DESCRIPTION OF THE DRAWINGS

Figure 1 shows a top view of a diaper in flat state suitable for being used with features of the present invention, but not embodying the present invention.
Figure 2 shows a cross-sectional view of the diaper of Figure 1 taken at the section line 2-2.
Figure 3 shows a top view of a diaper of the present invention in flat state.
Figure 4 shows a perspective view of the diaper of Figure 3.
Figure 5 shows a schematic sectional view of a barrier element, which is taken along a sectional cut in the longitudinal direction.
Figure 6 shows a schematic sectional view of another embodiment of a barrier element, which is taken along a sectional cut in the longitudinal direction.
Figure 7 shows a schematic sectional view of yet another embodiment of a barrier element, which is taken along a sectional cut in the longitudinal direction.

### DETAILED DESCRIPTION OF THE INVENTION

"Diaper" is used herein, to refer to absorbent articles to be placed about the wearer's lower torso, and include infant (baby and toddler) diapers and adult diapers, and it includes so-called diapers with fasteners, to be fastened around the lower torso, and pant-type diapers.

"Front region" (A) and 'back region' (C) are used herein to refer to the two regions, which are in use, respectively, the closest to the front of the wearer and the back of the wearer, each spanning the transverse dimension of the diaper or diaper portion or element thereof, e.g. elastic cuff or topsheet, and each region having a longitudinal dimension that is (exactly or about or around) one fourth of the longitudinal dimension of the diaper or diaper portion or element thereof, e.g. elastic cuff or topsheet.

"Crotch region" (B) is used herein to refer to the region positioned between the front region and the back region, having also a longitudinal dimension that is (exactly or about or around) one half of the longitudinal dimension of the diaper, diaper portion or element thereof, e.g. elastic cuff or topsheet.

"Longitudinal" is used herein to refer to the direction which is running substantially parallel or exactly parallel to the longitudinal centerline (Y) of the diaper or diaper portion or topsheet, which may be the machine direction (MD) of the process.

"Lateral" or "transverse" is used herein to refer to the direction which is substantially perpendicular or exactly perpendicular to the longitudinal centerline (Y) of the diaper or diaper portion or topsheet, which may be the cross-machine direction of the process (CD).

"Z-direction" is used herein to refer to the direction perpendicular to the longitudinal direction and perpendicular to the transverse or lateral direction.

"Longitudinal dimension", "transverse dimension" or "Z-dimension" is used herein to refer to the dimension of the diaper, diaper portion or element thereof, e.g. elastic cuff or topsheet, which is measured respectively in the longitudinal direction, transverse direction or Z-direction of the diaper or diaper portion or element thereof.

"Substantially perpendicular" or "substantially parallel" is used herein to refer to directions within 30° or 20° or 10° or 5° from the exact perpendicular or parallel direction, unless stated or specified otherwise.

As used herein, "along" means 'at least partially substantially parallel to and adjacent to'.

As used herein, "comprising" can also encompass "consisting of' or being "made of".

"Relaxed" or "relaxed state" or "contracted" or "contracted state" is used herein to refer to the state of the diaper or diaper portion wherein no forces are applied to respectively the diaper or diaper portion.

"Flat state" is used herein to refer to the state of the diaper when the diaper is laid out flat onto an even horizontal surface.

"Distal edge" is used herein to refer to the longitudinally extending edge of an elastic cuff which in use is closer to the wearer's body than the other longitudinally extending edge of the same elastic cuff.

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The invention will be further explained with reference to the figures. Additionally, the invention will be explained with regard to certain of its features. The invention relates to a diaper comprising *inter alia* a topsheet, a backsheet, longitudinally extending elastic cuffs, referred to also as barrier leg cuffs, elastic waist features and a bridge element. These elements will now first be described in some further detail.

### Barrier element

According to the present invention the diaper comprises a barrier element. This barrier element generally has the purpose to form a barrier between a front region and rear region of the diaper. Such a barrier is designed to be impermeable in particular for fecal material. Hence fecal material received in a rearward area will not or not freely travel to the front of the diaper.

It is useful therefore to position the barrier element in the crotch region of the diaper. For example, the barrier element may be positioned such that it divides the topsheet into a front and a back section, the front section and the back section being of equal length. Alternatively the front section might be slightly shorter than the back section, for example 10%, 15% or 20% shorter, but less than 35% shorter.

While in some embodiments more than one barrier element could be provided, for example two barrier elements, it is normally sufficient to provide one barrier element. One barrier element clearly devides the diaper into a front receiving section and a rear receiving section. An intermediate section, which could be created by providing two barrier element is often not required. If, however, more than one barrier element is provided, it can be useful to provide all barrier elements in the crotch region.

According to the present invention the barrier element extends in three dimensions. This is to say, that the barrier element has an essential extension in three dimensions. Herein, an essential extension denotes an extension given by more than the thickness dimension of the material, typically a sheet of material, used to provide the barrier element or its wall element or its bridge element.

As the barrier element extends in three dimensions it has a shape in the longitudinal plane of the diaper. Therefore in a sectional view taken along the longitudinal plane of the diaper the bridge element will assume a certain two dimensional form. Now, according to the present invention this respective two dimensional form should be symmetric. Therefore the shape will be symmetric about at least one transversal plane. The term transversal plane herein denotes a plane which is perpendicular to the longitudinal plane and which stands essentially upright one a flat surface which essentially coextensive with the diaper, when the diaper is in a flat position.

As the bridge element as a whole is symmetrical about at least one transversal plane, both the wall element and the bridge element are symmetrical about the respective transversal plane.

In useful embodiments of the present invention, however, already the wall element or the bridge element in themselves are symmetrical about other symmetric elements, such as other planes or axes or points. For example the bridge element could be symmetrical about another plane which is vertical to the longitudinal plane. This plane could also be vertical to the transversal plane and hence could be described as horizontal plane. Moreover, the bridge element could be symmetrical about an axis which is perpendicular to the longitudinal plane and which could also lay in the plane of the above mentioned horizontal axis.

Likewise, the wall element can have a shape which is symmetrical about a plane, which is perpendicular to the longitudinal plane. Again, the respective plane could be a horizontal plane. Moreover, the wall element could be symmetrical about an axis which is perpendicular to the longitudinal plane. Useful shapes could also be symmetrical about one central point, for example the shape could be that of a cubus or the like.

The existence of such further symmetry elements and generally providing highly symmetrical bridge elements and/or wall elements can be aesthetically pleasing. Moreover there can be important benefits for the manufacturing process. Generally, the wall element and/or the bridge elements are provided from materials which are supplied in wound form. Respective wound packages have to be unwound. Once a package is used up, the next package of material is to be fed to the manufacturing line. This is often done by operators. It is therefore desirable to have processes which can be easily executed and which, should they not be executed accurately, still work well. If the materials for the bridge element and/or the wall element can be fed to the manufacturing line such that for example a 180° turn (unwinding the material "upside down") does not negatively effect the process.

It can be useful to provide materials to the manufacturing line from different directions. In this regard, it can also be useful to have a relatively compact machine for unwinding different packagings of material from different directions. One example of a compact machine for unwinding multiple strength of material is given in WO 2013/095797 A1, which is incorporated herewith by reference. If materials are provided to the manufacturing line from different direction and especially if they are provided in a complex feeding pattern, it is beneficial if one particular sheet of material or element can be fed in different orientations, for example upside down or (also otherwise) turned by 180° or even turned by only 90° or the like.

The barrier element can comprise at least two separate elements, a wall element and a bridge element. Both elements, especially with a view to their desirable properties and with the view to useful materials for such elements will be discussed further. Generally, it can be said that the wall element stands up above and raises from the topsheet. Therefore, the wall element will have a proximal end, which is generally adjacent to the topsheet and a distal end which is opposite the proximal end and hence remote from the topsheet. The wall element largely provides the barrier function of the barrier element.

The barrier element further comprises a bridge element. The bridge element can usefully be positioned at the distal end of the wall element. The bridge element can contribute to the structural integrity of the barrier element. The bridge element can also readily provide attachment points for the attachment of the barrier element to other elements of the diaper, for example for the attachment to the longitudinally extending elastic cuffs.

The wall element will essentially extend perpendicular to the topsheet. Thereby, the wall element can efficiently provide a barrier function. Hence, the wall element will define a rearward barrier plane. Such a plane is a plane standing perpendicular on the topsheet and comprising the most rearwardly oriented point of the wall element. According to the present invention the bridge element comprises at least one element which is positioned rearward of this rearward barrier plane.

In some useful embodiments of the diaper several elements of the bridge element are positioned rearwardly of the rearward area plane and in other useful embodiments of the present invention all elements of the bridge element are positioned rearward of the rearward barrier plane.

The bridge element can be provided such that it is integral with at least a portion of the wall element at the distal end. It is also useful to provide the bridge element and at least the top portion of the wall element (or alternatively the full wall element from one sheet of material). It is useful that this sheet of material is a non-woven. The sheet of material can also be a film material or a combination of a film material and a non-woven material. The sheet of material can be provided as a non-woven -film - non-woven laminate. Different types of non-woven can usefully provide this sheet of material. It has been found that a spunbond - meltblown - spunbond non-woven is useful. This spunbond non-woven or another non-woven can also form part of a laminate together with other non-woven or film materials or other layers. Such other layers may in particular comprise an elastic material.

In other embodiments of course, the bridge element and the wall element are separate elements. They may be attached or in some cases also not be attached.

It can also be useful that the bridge element also comprises one element or several elements which are positioned in front of the rearward barrier plane. In one embodiment of the present invention the bridge element can form a T-shape in a sectional view along the longitudinal plane. In such an embodiment hence, the bridge element would comprise elements which are positioned in front of the rearward barrier plane and would comprise elements which are rearward of the rearward barrier plane. The respective T-shape can be fully symmetrical or can be essentially symmetrical. Also asymmetrical shapes can be useful.

### Bridge element

The bridge element may have a transverse dimension in relaxed state of from 2.5 cm, or from 3.0 cm, or from 4.0 cm, or from 5.0 cm, to for example 20.0 cm, or to 15.0 cm, or to 10.0 cm, or to 8.0 cm or less. The transverse dimension is to be taken between attachment points, normally attachment points to the cuffs.

The bridge element may have an average longitudinal dimension in relaxed state of at least 2.0 mm, or at least 4.0 mm, or at least 5.0 mm to less than 50.0 mm, 25.0 mm, 20.0 mm, 15.0 mm, 12.0 mm or less than 10.0 mm.

If the bridge element is provided in the form of the strip, the above longitudinal and transverse dimensions are those of that strip.

It is useful to position the bridge element in the crotch area of the diaper, for example in the center thereof and a bit closer to the front region of the diaper. The distance from the front edge of the diaper to the edge of the bridge element facing the front edge of the diaper may therefore be chosen to be 25% to 50% or 30 % to 45% of the to total length of the diaper taken from the front edge to the rear edge of the diaper.

The bridge element is elastically extensible in at least the transverse direction, i.e. is able to extend upon application of a force. The bridge element may also be elastically extensible in the longitudinal direction.

The bridge element may be made of an elasticated sheet material, rendering the bridge element elastically extensible in the transverse direction at least.

"Elasticated" when used herein for the bridge element means that it is made of an inelastic sheet material that has been provided only in a specific area or in specific areas, i.e. the elasticated area(s), with an elastic material, e.g. one or more elastic bands or strands.

One or more portions of the bridge element may be elasticated by application of one or more elastic band(s) or strand(s) extending substantially in transverse direction to a sheet material that may be inelastic e.g. an inelastic nonwoven sheet.

The nonwoven sheet material may be made of polyolefins known in the art, such as polyethylene and/or polypropylene, made into fibers, including bicomponent fibers that are then made into a nonwoven sheet. The nonwoven sheet material may be a necked nonwoven. The nonwoven sheet material may be a meltblown nonwoven or spunbond nonwoven or carded nonwoven; it may be a laminate thereof; for example it may be a laminate of spunbond or carded layer or layers and meltblown nonwoven layer(s). Generally, it may be provided as a non-woven-film - non-woven laminate.

The bridge element material may be a barrier material; it may be hydrophobic. It can also be provided as a hydrophilic material made hydrophobic with a hydrophobic surface coating, such as known in the art, for example a wax or a hydrophobic surface coating comprising one or more silicone polymers or fluorinated polymers. The bridge element may also be treated with a lotion.

### Bridge element material

The bridge element comprises a bridge element material. The bridge element may be essentially or fully provided by the material. The material can be a non-woven or a laminate, as mentioned above. Bridge element materials fulfilling certain criteria, as expressed below, have been found to work best. Certain strain measurements are therefore useful for identifying a beneficial material.

In use, the elongation of the bridge element material is not substantial. For example relevant elongation values can be 10 %, 20 %, 30 %, 40 %, 50 % or 70 % elongation. Often they are 30% or less. For example "10% elongation" means that a bridge element of length of 5 centimeters will have a length of 5.5 centimeters in the elongated state. Such as moderate enlongation however should induce sufficient restoring forces which can be expressed as it strength at 50 % elongation. This strength should be at least 0.2 N (Newton) or 0.5 N or 1 N in absolut terms. There is normally no requirement for the respective strength being greater than 30 N (Newton) or 20 N or 10 N. If the "restoring forces" of the bridge element materials are too strong, they may lead to the "restoring" of the longitudinal extended cuff to an undesired position or to the application of too high a force to the body of a wearerss.

It should also be considered that not only the restoring force in absolut terms is important, but the restoring force per width of material. In one aspect, it would be costly to employ a material which has a very high restoring force per centimeter of material width. Such a requirement would also limit the choice of suitable materials, in particular of suitable non-woven materials. In another aspect, the material must have a minimal restoring force per centimeter width of material. For good fit of the diaper, the bridge element must be placed in the perineal area. Especially for an infant this area is very small and hence the width available for the bridge element is limited. Therefore the bridge element should not only have a minimal restoring force in absolut terms (N) but should also have a minimal restoring force in per width unit (N per 10 mm). For a given diaper the restoring force is to be measured in the transverse direction, hence it is the force which is active in pulling the two longitudinally extending cuffs together. The respective width of the bridge element, for example the width of a strip of the bridge element material is to be measured with respect to the longitudinal extension of the diaper. Hence, the strength at 50 % elongation is to be understood as strength a newton in a first direction per distance in a second direction, this second direction being perpendicular to the first direction.

In a further important aspect, it should be considered in which temperature range the respective strength is present. A diaper is typically stored at room temperature which is normally around 20°C. Hence, when the diaper first contact the body of the wearer, the diaper will typically be at a temperataure of 20°C. A typical body temperature however is around 37°C. Hence the diaper will slowly warm up and might reach temperatures of up to 37°C. Often, a diaper after longer wearing periods reaches a temperature of 34°C. Hence the strength at elongation is of interest in context with a given temperature range. Ideally, the named values should be reached over the whole temperature range from 20°C to 37°C. For some embodiements it may be enough that the respective ranges are reached at 20°C or 34°C or 37°C.

In a further aspect it is important, that the bridge element material has a good elastic recovery. A material having a good elastic recovery will return to almost its initial shape after being elastically extended and being allowed to recover from this extension. This can be easily tested for extension in one linear direction. For example, a strip of material of a length of 10 cm may be stretched to a length of 15 cm under a given load. When the load stops to act on the strip, the strip may return to a length of 11 cm. Hence, the length by which the sample has recovered would be 4 cm. The length by which the sample was initially stretched in this example is 5 cm. Generally the elastic recovery rate is defined as the ratio between the length by which the sample has recovered versus the length by which the sample was strechted. This ratio is multiplied by 100 and expressed as a percent value for more conveniened reporting. In the example above, the elastic recovery rate would be optained by deviding as 4 cm / 5 cm x 100 = 80 %.

Especially in view of the repeated elastic stretching and elastic recovery a material with a elastic recovery of at least 70 % or 80 % or 90 % is preferred.

It is to be considered, that a material may have a different elastic recovery when in a dry state versus the wet state. Given the humid and sometimes wet ambient of the diaper, elastic recovery in the wet state should be considered. Preferred materials have the above elastic recovery values both in the dry state and in the wet state.

It should be noted that a bridge element comprising a bridge element material can be a bridge element consisting of and solely provided from the bridge element material.

### Wall element and wall element material

The barrier element comprises a wall element. The wall element consists of or comprises a wall element material.

The wall element material can be attached to the bridge element material but it is also possible to provide the bridge element and the wall element from a unitary sheet of material. Also therefore, the strength at 50 % elongation values and the elastic recovery values specified for the bridge element materials are also relevant for the wall element sheet material.

It should be noted that a wall element comprising a wall element material can be a wall element consisting of and solely provided from the wall element material.

Further it should be noted, that providing a barrier element in the form of two designated elements, namely a bridge element and a wall element is associated with a number of advanteages. Within the present invention a diaper is useful, wherein the bridge element material and / or the wall element material is provided as a fabric which is pre-treated by a process for imparting high-elastic recovery. One suitable process is disclosed in US 3,637,427.

Within the present invention a diaper is useful, wherein the bridge element material and / or the wall element material is provided as an elastic non-woven fabric containing a long elastomeric fiber and a nonelastomeric fiber. One suitable nonwoven fabric is disclosed in US 2004/0067710 A1.

The bridge element material and / or the wall element material may comprise an elastic film or a nonwoven sheet material or a laminate of an elastic film and a nonwoven sheet material. The nonwoven sheet material of the laminate can be positioned such that it is in contact with the skin of the wearer. Such a configuration of the laminate may provide more comfort to the wearer than when the elastic film is directly in contact with the skin of the wearer.

The bridge element material and /or wall element material may comprise a nonwoven sheet material that has a substantially uniform elastic behavior due to the application (e.g. substantially uniformly) of an elastic material to the nonwoven sheet material, in a way that it provides elasticity at least in transverse direction to the nonwoven sheet material; for example, elastic material may be sprayed or extruded onto a nonwoven sheet material, e.g. homogeneously or in a pattern such as stripes in transverse direction.

### TEST METHODS

### Preparation of samples

Samples to be used for any test described hereinbelow should normally prepared as follows.

Before obtaining the specimen from the diaper, the diaper is kept in a relaxed state at ambient temperature (22°C +/- 5°C) for at least 24 hours.

The bridge element material or wall element material is cut to obtain a rectangular specimen having a transverse dimension (length) of 25 mm centered on the longitudinal centerline Y of the diaper and a longitudinal dimension (width) of 2 mm, both transverse and longitudinal dimensions being measured in relaxed, contracted state of the specimen when the specimen is laid out flat onto an even horizontal surface. The transverse dimension and longitudinal dimension of the specimen are measured parallel to respectively the transverse dimension and longitudinal dimension of the bridge element material. As the cuff attachment areas might have a reduced elasticity, as they might, for example, comprise an adhesive, the specimen should be free of such attachment areas.

Before starting the test (details below), each specimen and the equipment must equilibrate at the appropriate test temperature, e.g. 37°C (+/- 1°C), for at least 1 hour.

### Equipment and test procedures

Elastic recovery should be measured as disclosed in US 3,637,427.

Stength at 50% elongation should be measured as disclosed in US 2004/0067710A1, specifically in paragraphs [0082] and [0083]. In deviation from the respective test method (and the general sample preparation above) it is preferred however to use a test piece which is 10 mm wide and 30 mm long. Allowing for 5 mm clamp length at each end, the free length of the specimen is 20 mm. The rate of stretch should be 30 mm/min.

Generally, the method can be used with different test piece sizes. Relevant parameters are to be adapted to the test piece size, e.g. rate of stretch is to be reduced proportionally with sample length. Therefore, where advantageous, the use of a larger test piece can be considered, when results are adapted accordingly.

If slippage of the test piece relative to the clamp is observed, it should be considered to increase clamping pressure and /or the roughness of the clamping surfaces and/or other methods known in the field for suppressing slippage.

Should the test equipment named in these patent documents not be available it can normally be replaced, at least for initial testing, by the equipment specified below. If not in contrast to the disclosure in the above patent documents, the procedure for use of tensile testers given below should be followed.

Suitable instruments for this test include tensile testers commercially available from MTS Systems Corp., Eden Prairie, Minn. (e.g. Alliance RT/1 or Sintech 1/S) or from Instron Engineering Corp., Canton, Mass. or from Zwick/Roell, Kennesaw, Georgia/Ulm, Germany or Shimadzu (e.g. via Shimadzu Deutschland GmbH, Albert-Hahn-Str. 6, 47269 Duisburg, e.g. model Autograph AG-G).

The tester is typically equipped with a grip face set in the upper and lower grips, where each set has one rubber-coated face (80 A shore hardness) and one contact line face (metal), with a screw mechanism that engages the contact line face into the rubber-coated face to prevent slippage. The length of the upper and lower grips is at least as long as the clamped length of the specimen to be tested, the grip length being the dimension of the grip parallel to the length of the specimen. A load cell is used so that the maximum load measured is within 10 - 90% of the maximum capacity of the load cell. The instrument is calibrated according to the manufacturer's specification.

The force reading on the instrument is zeroed to account for the mass of the fixtures and grips. The specimen is mounted into the grips with no slack and the force measured is between 0.00 N and 0.01 N. The data acquisition frequency is typically 50Hz or 100 Hz; the force, time and engineering strain data are acquired during all segments of the hysteresis tests.

The specimen is mounted with 10 mm of its width in relaxed, contracted state between the lines of contact of the grips (gauge width). The clamped length of the specimen is clamped in the grips, which should be 5 mm at each side. The remaining length of the specimen (which equals the length of the specimen minus the clamped length) is excluded from the grips.

### Barrier leg cuffs

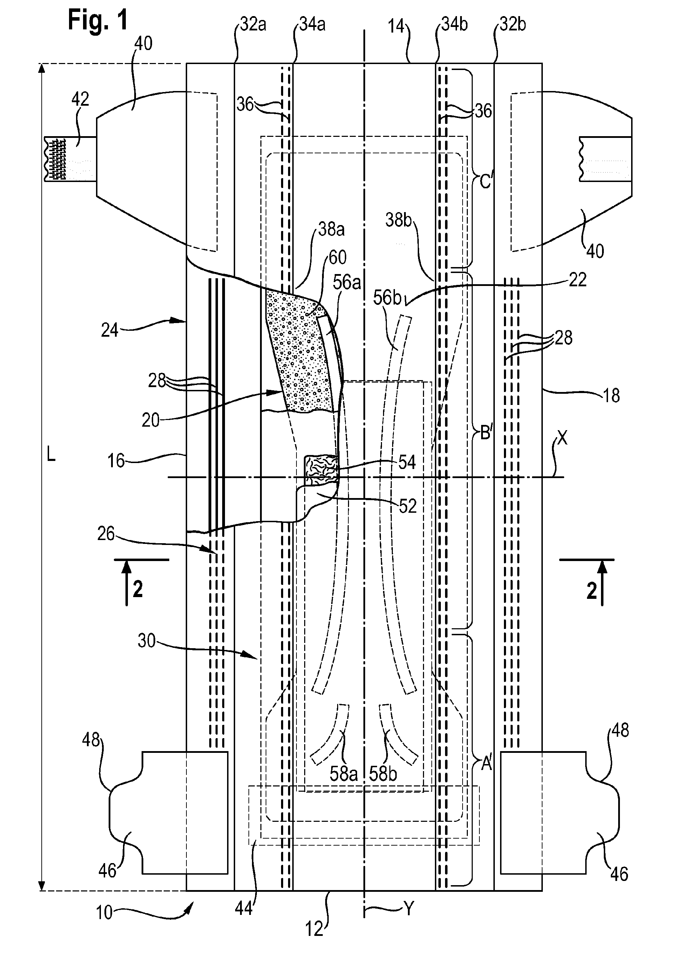

The absorbent article comprises a pair of barrier leg cuffs, also referred to as longitudinally extending elastic cuffs. The barrier leg cuffs can be formed from a piece of material, typically a nonwoven, which is partially bonded to the rest of the article so that a portion of the material, the barrier leg cuffs, can be partially raised away and stand up from the plane defined by the topsheet when the article is pulled flat as shown e.g. in Fig. 1. The barrier leg cuffs can provide improved containment of liquids and other body exudates approximately at the junction of the torso and legs of the wearer. The barrier leg cuffs extend at least partially between the front edge and the back edge of the diaper on opposite sides of the longitudinal axis. The barrier leg cuffs are delimited by a proximal edge joined to the rest of the article, typically the topsheet and/or the backsheet, and a free distal edge, which is intended to contact and form a seal with the wearer's skin. The barrier leg cuffs are joined at the proximal edge with the chassis of the article by a bond which may be made for example by gluing, fusion bonding or combination of known bonding means. The bond at the proximal edge may be continuous or intermittent. The side of the bond closest to the raised section of the leg cuffs delimits the proximal edge of the standing up section of the leg cuffs.

The barrier leg cuffs can be integral with the topsheet or the backsheet, or more typically be formed from a separate material joined to the rest of the article. Typically the material of the barrier leg cuffs may extend through the whole length of the diapers but is "tack bonded" to the topsheet towards the front edge and back edge of the article so that in these sections the barrier leg cuff material remains flush with the topsheet. Each barrier leg cuff 34 may comprise one, two or more elastic strings 36 close to this free terminal edge to provide a better seal.

Each cuff may have an elastic tension of at least 20 grams (0.2N) and up to 100 grams (1.0N), or up to 50 grams (0.5N), when extended to a 95% or 80% extension strain.

The cuffs or cuff web material may comprise a nonwoven sheet material. The same nonwoven sheet materials as the ones used to make the bridge element may be used. The nonwoven sheet material may be a nonwoven barrier sheet material that is liquid impermeable, as known in the art, including for example nonwoven laminate(s) with one or more spunbond layers and/or carded layers, and one or more meltblown layers. The fibers used to form the nonwoven sheet material may be selected from polypropylene fibers, polyethylene fibers, bicomponent fibers, nano-fibers and any combinations thereof.

The cuffs may have any suitable dimensions, for example depending on the diaper dimensions. They may extend about the full length of the diaper. They may have a transverse dimension perpendicular to longitudinal centerline Y of the diaper of for example at least 30 mm, or for example at least 40 mm.

In addition to the barrier leg cuffs, the article may comprise gasketing cuffs, which are joined to the chassis of absorbent article, in particular the topsheet and/or the backsheet and are placed transversely outwardly relative to the barrier leg cuffs. The gasketing cuffs can provide a better seal around the thighs of the wearer. Usually each gasketing leg cuff will comprise one or more elastic string or elastic element comprised in the chassis of the diaper for example between the topsheet and backsheet in the area of the leg openings.

US3,860,003 describes a disposable diaper which provides a contractible leg opening having a side flap and one or more elastic members to provide an elasticized leg cuff (a gasketing cuff). US4,808,178 and US4,909,803 issued to Aziz et al. describe disposable diapers having "stand-up" elasticized flaps (barrier leg cuffs) which improve the containment of the leg regions. US4,695,278 and US4,795,454 issued to Lawson and to Dragoo respectively, describe disposable diapers having dual cuffs, including gasketing cuffs and barrier leg cuffs. All or a portion of the barrier leg and/or gasketing cuffs may be treated with a lotion.

### Topsheet

The diaper comprises a topsheet.

It should be understood that the topsheet herein may be an individual topsheet or a topsheet that is part of a topsheet web, whereby such web is then subsequently divided into a multitude of individual topsheets. For the purpose of the invention, when referred to topsheet, this shall include a topsheet web, respectively, unless stated otherwise. The same applies for the backsheet and absorbent core (that may be a backsheet web or absorbent core web), as referred to herein.

The topsheet of the diaper herein may be made of any suitable material known in the art, provided it allows urine to pass. Hereto, it may be made of a urine permeable material, including hydrophilic material, or material treated to be hydrophilic. It may be an apertured topsheet which comprise apertures to allow urine to pass to the absorbent core under the topsheet;

The topsheet may be either hydrophilic or hydrophobic.

Preferred topsheet materials are nonwoven materials, including laminates and/or materials with apertures, such as apertured films, aperture formed films.

The topsheet, or for example only the crotch and/or back region thereof, may comprise a skin care composition, e.g. a lotion, as known in the art.

### Fastening system

The absorbent article may include a fastening system. The fastening system can be used to provide lateral tensions about the circumference of the absorbent article to hold the absorbent article on the wearer as is typical for taped diapers. This fastening system is not necessary for training pant article since the waist region of these articles is already bonded. The fastening system usually comprises a fastener such as tape tabs, hook and loop fastening components, interlocking fasteners such as tabs & slots, buckles, buttons, snaps, and/or hermaphroditic fastening components, although any other known fastening means are generally acceptable. A landing zone is normally provided on the front waist region for the fastener to be releasably attached. Some exemplary surface fastening systems are disclosed in US 3,848,594, US4,662,875, US 4,846,815, US4,894,060, US4,946,527, US5,151,092 and US 5,221,274 issued to Buell. An exemplary interlocking fastening system is disclosed in US6,432,098. The fastening system may also provide a means for holding the article in a disposal configuration as disclosed in US 4,963,140 issued to Robertson et al.

The fastening system may also include primary and secondary fastening systems, as disclosed in US4,699,622 to reduce shifting of overlapped portions or to improve fit as disclosed in US5,242,436, US5,499,978, US5,507,736, and US5,591,152.

As part of the fastening system, the absorbent article may comprise front ears and back ears as is known in the art. The ears can be integral part of the chassis, for example formed from the topsheet and/or backsheet as side panel. Alternatively, they may be separate elements attached by gluing and / or heat embossing or pressure bonding. The back ears are advantageously stretchable to facilitate the attachment of the tabs on the landing zone and maintain the taped diapers in place around the wearer's waist. The back ears may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

### Elastic waist feature

The absorbent article may also comprise at least one elastic waist feature (not represented) that helps to provide improved fit and containment. The elastic waist feature is generally intended to elastically expand and contract to dynamically fit the wearer's waist. The elastic waist feature preferably extends at least longitudinally outwardly from at least one waist edge of the absorbent core 28 and generally forms at least a portion of the end edge of the absorbent article. Disposable diapers can be constructed so as to have two elastic waist features, one positioned in the front waist region and one positioned in the back waist region. The elastic waist feature may be constructed in a number of different configurations including those described in US4,515,595, US4,710,189, US5,151,092 and US 5,221,274.

If should be noted, that the design (including the dimensions) of the cuffs, also the gasketing cuffs, and of the elastic waist feature and the fastening system has an influence on the overall fit of the diaper when worn and therefore on the position and functioning of the bridge element. Where elastics are used in the various portions of the diaper, their elastic behavious also has such an influence.

### Relations between the layers

Typically, adjacent layers and components will be joined together using conventional bonding method such as adhesive coating via slot coating or spraying on the whole or part of the surface of the layer, or thermo-bonding, or pressure bonding or combinations thereof. This bonding is not represented in the Figures (except for the bonding between the raised element of the leg cuffs with the topsheet) for clarity and readability but bonding between the layers of the article should be considered to be present unless specifically excluded. Adhesives may be typically used to improve the adhesion of the different layers, for example between the backsheet and the core wrap. The glue may be any standard hotmelt glue as known in the art.

### Method of making the article

The absorbent articles of the invention may be made by any conventional methods known in the art. In particular the articles may be hand-made or industrially produced at high speed

### Backsheet

The backsheet of the diaper may be liquid impervious, as known in the art. The liquid impervious backsheet preferably comprises a thin plastic film such as a thermoplastic film, for example having a thickness of about 0.01 mm to about 0.05 mm. Suitable backsheet materials comprise typically breathable material, which permit vapors to escape from the diaper while still preventing exudates from passing through the backsheet. Suitable backsheet films include those manufactured by Tredegar Industries Inc. of Terre Haute, IN and sold under the trade names X15306, X10962 and X10964.

The backsheet, or any portion thereof, may be elastically extendable in one or more directions. The backsheet may be attached or joined to a topsheet, the absorbent core, or any other element of the diaper by any attachment means known in the art. It may be highly preferred that the longitudinal side edges of the topsheet and backsheet are directly attached to one another.

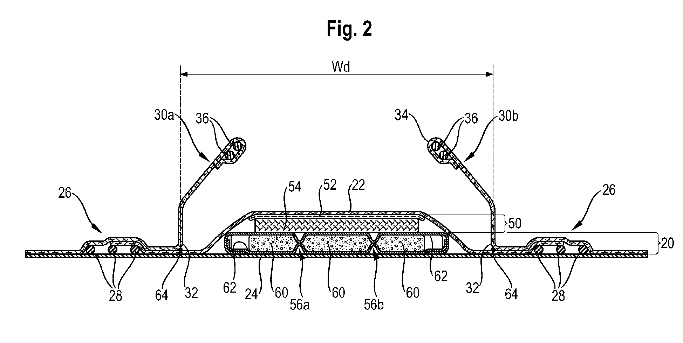

The essential elements of diaper 10, for which the present invention can be useful, are shown in Figures 1 and 2. Notably, the respective diaper does not already include the features of the present invention, however, serves to illustrate the benefits achievable by the present invention, once the invention is used for a diaper such as shown in Figure 1 and Figure 2.

The diaper 10 comprises a front edge 12 and a rear edge 14. The diaper also has a left side edge 16 and a right side edge 18. For the purpose of absorbing liquids, the diaper comprises absorbent core 20. The absorbent core 20 is positioned between a topsheet 22 and a back sheet 24. The topsheet 22 is positioned on the wearer facing side of the diaper 10 and will allow, at least in one portion, the passage of liquids. The backsheet 24, however, is normally liquid proof (but may be vapor permeable).

The diaper 10 comprises gasketing cuffs 26, arranged on the wearer facing side, which is shown in the view of Figure 1. The gasketing cuffs 26 comprise elastics 28. These elastics 28 are normally arranged between topsheet 22 and backsheet 24. Alternatively, the elastics 28 can be provided between an extention sheet of the barrier leg cuffs 30 and the backsheet 24. The extension sheet can be joined to or intregral with the barrier leg cuff(s). The elastics 28 can also be provided in the form of lines of elastic adhesives.

The diaper 10 also comprises barrier leg cuffs 30 (also referred to as "BLCs" or longitudinally extending cuffs). These barrier leg cuffs 30 are readibly visible on the barrier facing side of the diaper, which is shown in the view of Figure 1. The barrier leg cuffs 30 exhibit a proximal edge 32 and a distal edge 34. The proximal edge 32 extends along the longitudinal direction of the diaper. Typically, the barrier leg cuffs 30 are attached to the diaper 10 at least along their proximal edge 32. Normally, at least in the vicinity of front edge 12 and rear edge 14 further attachment areas are present. The barrier leg cuffs 30 also each have a distal edge 34, which also extends in the longitudinal direction.

The barrier leg cuffs 30 are equiped with elastics 36 positioned adjacent to distal edge 34. By means of these elastics 36, the barrier leg cuffs 30 can be brought in good contact with a wearer and will remain in contact with the wearer while the diaper 10 is worn. A portion of the barrier leg cuffs 30 is normaly not attached to other portions of the diaper 10 and therefore forms a free flap 38. Such a free flap 38 is typically provided at least in the crotch portion B' of the barrier leg cuff 30. Typically, two longitudinally extending barrier leg cuffs are provided on either side of a diaper. As shown in Figure 1, these are barrier leg cuffs 30a and 30b. Each cuff has a respective proximal edge 32a, 32b and a respective distal edge 34a, 34b. Further, each cuff is provided with a free flap 38a, 38b. The barrier leg cuffs and in particular their free flaps 38a, 38b are provided in the form of mirror images and are centered about the longitudinal axis Y of the diaper.

The diaper 10 is also equipped with a fastening system. In the back region C of the diaper, the fastening system comprises back ears 40. The back ears 40 are provided with adhesive tapes 42. A landing zone 44 for the adhesive tapes 42 is provided in a region of the backsheet 24. When the diaper 10 is placed around the lower torso of a wearer, the back ears 40 co-operate with front ears 46. The front ears 46 comprise a tab portion 48.

Figure 2 provides a cross section view of the diaper as indicated by line 2-2 in Figure 1. This cross sectional view, provides more details about the structure of the core 20. Underneath topsheet 22, an acquisition distribution system 50 is provided. This system comprises an acquisition layer 52, which is placed adjacent to or in proximity of topsheet 22. Underneath the acquisition layer 52 a distribution layer 54, serving for the distribution of liquid, is arranged. Underneath the acquisition distribution system 50, core 20 is placed. As visible in this view, the core 20 comprises a number of channels 56. Also, several channels of different dimensions can be provided, such as further channel 58 as visible in Figure 1. Between such channels absorbent material 60 is arranged. The absorbent material 60 is covered by core wrap 62.

It is also readibly visible from Figure 2, that dedicated attachment means are provided at the proximal edge 32 of the barrier leg cuff 30. As shown, this attachment means can be provided in the form of a barrier leg cuff bond 64. A respective bond can be provided by a longitudinally extending line of glue.

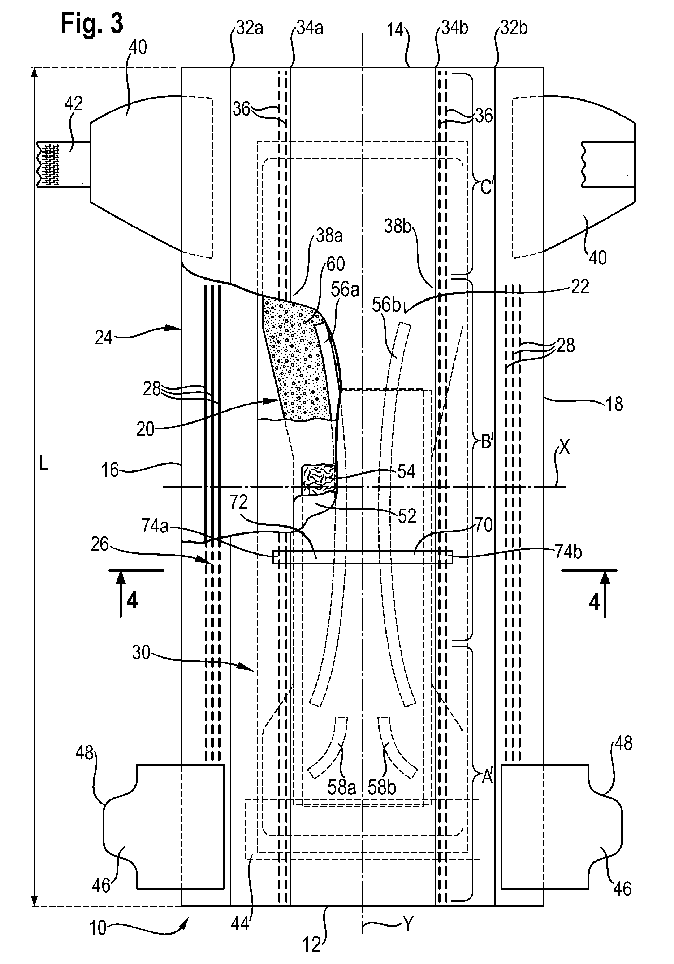

Figure 3 provides a top plane view onto the barrier facing side of a diaper 10 according to the present invention. In the central or crotch region B of the diaper and thereby the central or crotch region B' of the barrier leg cuffs, a bridge element 70 is arranged. The bridge element 70 is provided in form of a strip 72. This strip 72 is attached to each of the barrier leg cuffs 30a and 30b. The attachment is achieved in attachment areas 74a and 74b.

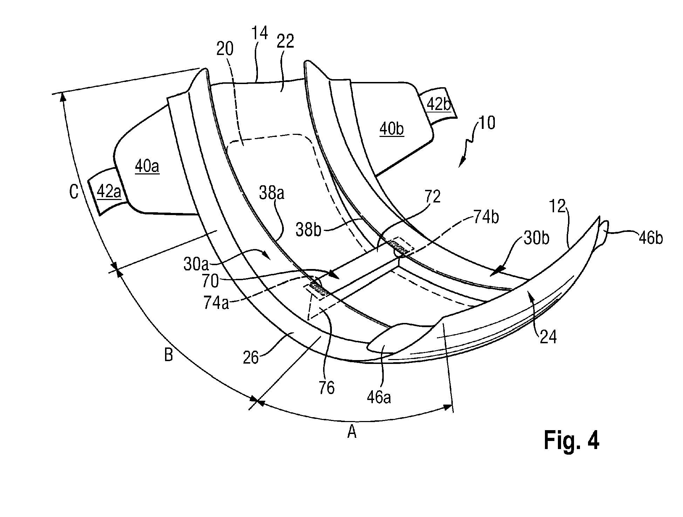

As shown in Figure 4, the barrier element 70 can comprise a bridge element 72 and a wall element 76. The bridge element 72 can be connected to the first longitudinally extending cuff 30a a first attachment point 74a. The bridge element 72 can be connected to the second longitudinally extending cuff 30b at second attachment area 74b. The attachment of the bridge element 72 to the respective cuffs gives the barrier element 70 a structural integrity. In partuclar it can be thereby ensured that the wall element 76 raises essentially perpendicularly above topsheet 22. Hence the proximal end of the wall element 76 is adjacent to a portion of topsheet 22. The opposing distal end of wall element 76, however, is adjacent to bridge element 72. It is often useful to provide an attachment between the distal portion of wall element 76 and bridge element 72. Alternatively, the bridge element 72 and the wall element 76, or at least portions thereof, can be provided from one sheet of material. In this fashion, the need to make an attachment between the two elements can be obsoleted.

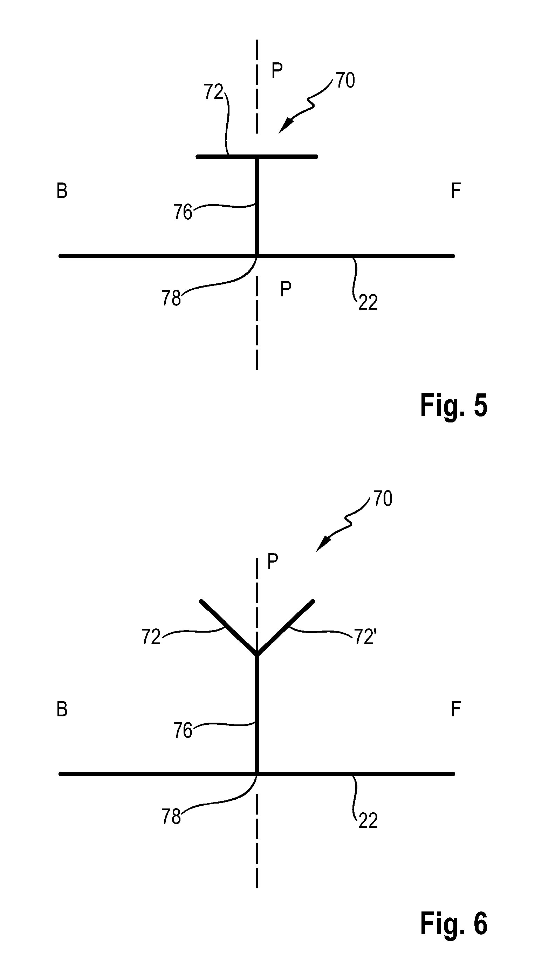

Figure 5 shows a schematic sectional view of a barrier element 70, which is taken along a sectional cut in the longitudinal direction. Barrier element 70 raises above topsheet 22. The wall element 76 is perpendicular to tophsheet 22. Wall element 76 is attached to topsheet at the attachment area 78. The proximal end of wall element 76 is attached to a portion of the topsheet 22 at attachment area 78. The opposite of wall element 76, the distal end is attached to bridge element 72.

As the sectional view is taken along the longitudinal direction the transversal direction and the transversal plane would stand perpendicular on the sheet of paper representing the figure. The respective plane is indicated as plane "P". The rearward area or backward area of the diaper (on the left side of plane P) is represented by the letter "B" and the front area of the diaper (at the right side of plane P) is represented by the letter "F".

As can be seen, the bridge element 70 is symmetrical about plane P. The wall element 76 is aligned with the plane and the bridge element 72 is positioned symmetrically relative to plane P. Therefore, both the wall element 76 and the bridge element 72 are symmetrical about plane P. The bridge element 72 is provided in the form of a stripe of material and overall, the barrier element forms a T-shape.

Figure 6 represents in a schematic way another embodiment of a barrier element 70. Also in this embodiment the wall element 76 stands essentially perpendicular on topsheet 22. The bridge element 72 is provided in another form. It has overall a V-shape. The V, however, is positioned such as the plane P is a mirror plane for the V. Therefore, also in this embodiment the barrier element overall is symmetrical about the transversal plane P.

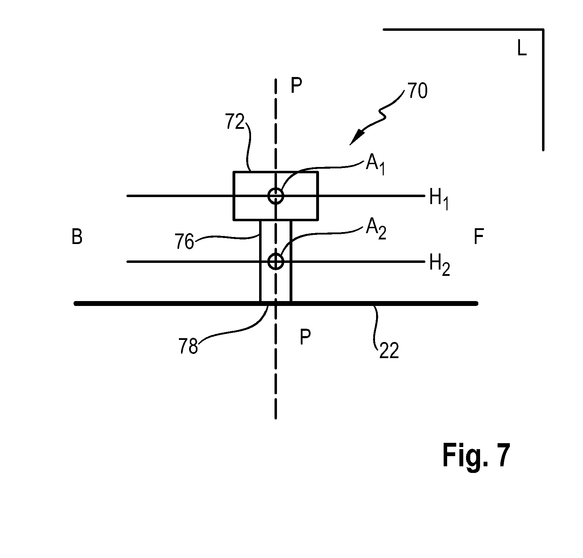

Figure 7 represents in a schematic way another embodiment of a barrier element 70. Also in this embodiment the wall element 76 stands essentially perpendicular on topsheet 22. The bridge element 72 is provided in another form. It has overall a V-shape. The V, however, is positioned such as the plane P is a mirror plane for the V. Therefore, also in this embodiment the barrier element overall is symmetrical about the transversal plane P. Again, the wall element 76 stands essentially perpendicular on topsheet 22. The bridge element 72 is provided on top of wall element 76. Both the bridge element 72 and the wall element 76 have a rectangular shape in this cross sectional view. Both elements together or in other words the bridge element 70 are symmetrical about the transversal plane P. However, botht the bridge element 72 and the wall element 76 have further symmetric element.

For example, bridge element 72 is symmetrical about plane H₁. Plane H₁ is a horizontal plane which stands perpendicular on transversal plane P and on longitudinal plane L. Moreover, the bridge element 72 is symmetrical about its central axis A₁.

Likewise, wall element 76 is symmetrical about plane H₂. Plane H₂ is a horizontal plane which stands perpendicular on transversal plane P and on longitudinal plane L. Moreover, the wall element 76 is symmetrical about its central axis A₂.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An infant or adult diaper (10) having a longitudinal centerline (Y) and a lateral centerline (X) perpendicular to the longitudinal centerline (Y), wherein the diaper (10) comprises a topsheet (22) and a backsheet (24) and positioned therein between an absorbent core (20), and a first longitudinally extending elastic cuff (30a) and second longitudinally extending elastic cuff (30b), the first and the second elastic cuff (30a, 30b) being positioned on either longitudinal side of the topsheet (22) and opposing each other, and wherein the diaper (10) has a crotch region (B), positioned in between a front region (A) and a back region (C) and the first and the second longitudinally extending elastic cuff (30a, 30b) each have a crotch region (B'), positioned in between a front region (A') and a back region (C'), these regions of the longitudinally extending elastic cuffs (30a, 30b) being positioned in the respective regions of the diaper (10), and the first longitudinally extending elastic cuff (30a) and the second longitudinally extending elastic cuff (30b) each have a proximal edge (32a, 32b), at which the respective longitudinally extending elastic cuff is attached to the diaper and the first longitudinally extending elastic cuff (30a) and the second longitudinally extending elastic cuff (30b) each have a distal edge (34a, 34b), at least the crotch region (B') of which is not attached to the diaper (10) and forms a free flap (38a, 38b) and wherein the diaper (10) comprises a barrier element (70) arranged in the crotch region (B), the barrier element (70) comprising at least a wall element (76) and bridge element (72), the barrier element (70) extending in three dimensions such that it has a shape in the longitudinal plane of the diaper, the shape being symmetrical about at least one transversal plane.

2. An infant or adult diaper (10) according to claim 1, wherein the bridge element (72) has a shape in the longitudinal plane of the diaper, the shape being symmetrical about at least one horizontal plane.

3. An infant or adult diaper (10) according to claim 1, wherein the wall element (76) has a shape in the longitudinal plane of the diaper, the shape being symmetrical about at least one horizontal plane.

4. An infant or adult diaper (10) according to claim 1, wherein the wall element (76) comprises a proximal end, which is generally adjacent to the topsheet (22) and a distal end opposite the proximal end and wherein the bridge element (72) is arranged at the distal end.

5. An infant or adult diaper (10) according to any one of the preceding claims, wherein the bridge element (72) is intregral at least with the portion of the wall element (76) at the distal end.

6. An infant or adult diaper (10) according to any one of the preceding claims wherein the bridge element (72) and a portion of the wall element (76) are provided from one sheet of material.

7. An infant or adult diaper (10) according to the preceeding claim wherein said sheet of material is a non-woven.

8. The diaper (10) according to any one of the preceding claims, wherein the barrier element (70) is made of or comprises a laminate, which comprises an elastic material or a non-woven material.

9. An infant or adult diaper (10) according to any one of the preceding claims wherein the bridge element (72) also comprises an element which is positioned in front of the rearward barrier plane.

10. An infant or adult diaper (10) according to any one of the preceding claims wherein at least two barrier elements are provided and all barrier elements are provided in the crotch region (B).

11. An infant or adult diaper (10) according to any one of the preceding claims wherein the barrier element (70) forms a T-shape in a sectional view along a longitudinal plane.

12. The diaper (10) according to any one of the preceding claims, wherein the bridge element (72) has a length, as measured in the transversal direction, from about 2.5 cm to about 20 cm.

13. An infant or adult diaper (10) according to any one of the preceding claims wherein the barrier element (70) is directly or indirectly attached to the first longitudinally extending elastic cuff (30a) and the second longitudinally extending elastic cuff (30b) and the barrier element (70) comprises a material, which has a strength at 50% elongation of at least 0,2 N per 10 mm and of no more than 10 N per 10 mm in a temperature range of 20° C to 37°C.

14. An infant or adult diaper (10) according to any one of the preceding claims wherein the wall element (76) is directly or indirectly attached to the first longitudinally extending elastic cuff (30a) in its crotch region (B') and the second longitudinally extending elastic cuff (30b) in its crotch region (B')

15. An infant or adult diaper (10) according to any one of the preceding claims wherein the bridge element (72) is directly attached to the first longitudinally extending elastic cuff (30a) and the second longitudinally extending elastic cuff (30b) and the bridge element (72) comprises a material, which has a strength at 50% elongation of at least 0,2 N per 10 mm and of no more than 10 N per 10 mm in a temperature range of 20° C to 37°C.
